# EUROPEAN PATENT SPECIFICATION

(11) **EP 4 166 940 B1**
(45) Date of publication and mention of the grant of the patent: **16.04.2025**
(21) Application number: 22202241.0
(22) Date of filing: 18.10.2017
(51) Int. Cl.: G01N 27/12

(54) **GAS SENSOR, METHOD OF MAKING THE SENSOR AND METHOD OF USING THE SENSOR**
GASSENSOR, VERFAHREN ZUR HERSTELLUNG DES SENSORS UND VERFAHREN ZUR VERWENDUNG DES SENSORS
CAPTEUR DE GAZ, PROCÉDÉ DE FABRICATION DU CAPTEUR ET PROCÉDÉ D'UTILISATION DU CAPTEUR

(30) Priority: 18.10.2016 US 201662409626 P
(43) Date of publication of application: 19.04.2023
(62) Divisional of application: 17791891.9
(73) Proprietor: Carrier Corporation, Palm Beach Gardens, FL 33418 (US)
(72) Inventor: SANKARRAJ, Anand Venkatesh, Minneapolis (US); FOLLETT, Gary, Minneapolis (US); FILLMORE, Robert L., Minneapolis (US); EVJU, Jon K., Minneapolis, Minnesota (US)
(74) Representative: Dehns

(56) References cited:
- EP-A1- 1 950 558
- JP-A- 2007 024 508
- US-A- 5 624 640
- YUANDA W ET AL: "Thin film sensors of SnO"2-CuO-SnO"2 sandwich structure to H"2S", SENSORS AND ACTUATORS B: CHEMICAL, ELSEVIER BV, NL, vol. 79, no. 2-3, 15 October 2001 (2001-10-15), pages 187 - 191, XP004305519, ISSN: 0925-4005, DOI: 10.1016/S0925-4005(01)00873-5
- GONG J ET AL: "Micromachined nanocrystalline silver doped SnO"2 H"2S sensor", SENSORS AND ACTUATORS B: CHEMICAL, ELSEVIER BV, NL, vol. 114, no. 1, 30 March 2006 (2006-03-30), pages 32 - 39, XP027971379, ISSN: 0925-4005, [retrieved on 20060330]
- R.S. NIRANJAN ET AL: "High H2S-sensitive copper-doped tin oxide thin film", MATERIALS CHEMISTRY AND PHYSICS, vol. 80, no. 1, 14 November 2002 (2002-11-14), Switzerland, Taiwan, Republic of China, pages 250 - 256, XP055437751, ISSN: 0254-0584, DOI: 10.1016/S0254-0584(02)00467-4
- NIRANJAN R S ET AL: "A novel hydrogen sulfide room temperature sensor based on copper nanocluster functionalized tin oxide thin films", SENSORS AND ACTUATORS B: CHEMICAL: INTERNATIONAL JOURNAL DEVOTED TO RESEARCH AND DEVELOPMENT OF PHYSICAL AND CHEMICAL TRANSDU, ELSEVIER BV, NL, vol. 85, no. 1-2, 20 June 2002 (2002-06-20), pages 26 - 32, XP004358217, ISSN: 0925-4005, DOI: 10.1016/S0925-4005(02)00046-1

## Description

### BACKGROUND

Gas sensors have been used in various applications such as process monitoring and control and safety monitoring. As the compounds can also be flammable or explosive, gas detection sensors have also been used for leak detection where such compounds are used or manufactured. Various types of sensors have been used or proposed, including but not limited to metal oxide semiconductor (MOS) sensors, non-dispersive infrared detector (NDIR) sensors, pellistor (pelletized resistor) sensors, high-temperature solid electrolytes that are permeable to oxygen ions, and electrochemical cells.

The above types of sensors have been used with varying degrees of success in the industrial or laboratory settings where they have been employed. However, many such sensors have limitations that can impact their effectiveness in demanding new and existing applications. For example, pellistor sensors are prone to false alarms due to cross-sensitivity. NDIR sensors have been used in low-volume applications, but can be difficult and expensive to manufacture to commercial tolerances. Electrochemical sensors rely on redox reactions involving tested gas components at electrodes separated by an electrolyte that produce or affect electrical current in a circuit connecting the electrodes. However, solid state electrochemical sensors can be difficult to implement for some materials. For example, solid state electrochemical sensors testing for combustible hydrocarbons may utilize solid electrolytes formed from ceramics such as perovskite, which can require high temperatures (typically in excess of 500°C) that render them impractical for many applications. Some electrochemical sensors that operate at lower temperatures (e.g., carbon monoxide sensors, hydrogen sulfide sensors) require the presence of water at the electrode/electrolyte interface for the electrochemical redox reactions, which can render them impractical for many applications.

MOS sensors rely on interaction between gas test components such as hydrogen sulfide or hydrocarbons with adsorbed oxygen on the metal oxide semiconductor surface. In the absence of the gas test components, the metal oxide semiconductor adsorbs atmospheric oxygen at the surface, and this adsorbed oxygen captures free electrons from the metal oxide semiconductor material, resulting in a measurable level of base resistance of the semiconductor at a relatively high level. Upon exposure to gas test components such as hydrogen sulfide or hydrocarbon, the gas test component interacts with the adsorbed oxygen, causing it to release free electrons back to the semiconductor material, resulting in a measurable decrease in resistance that can be correlated with a measured level of test gas component.

In view of the demanding requirements for gas sensors, there remains a need for new alternatives for various environments and applications.

### BRIEF DESCRIPTION

A gas-sensing element according to a first aspect of the invention is described in claim 1. This gas-sensing element comprises a body comprising a semiconductor that is a metal oxide of a first metal. This semiconductor is also referred to herein as a "metal oxide semiconductor" or "MOS". The gas-sensing element includes a gas-sensing surface over the body. The gas-sensing surface comprises metal oxide semiconductor of the first metal and a dopant comprising a second metal that is a transition metal and is different than the first metal. The gas-sensing element also includes an auxiliary component comprising: a metal chalcogenide disposed at the gas-sensing surface, wherein the gas-sensing element comprises a plurality of alternating deposits of the metal oxide semiconductor of the first metal and deposits of the second metal, disposed in the gas-sensing element between the body and the gas-sensing surface.

In any one or combination of the foregoing embodiments, the metal chalcogenide may comprise a metal sulfide.

In any one or combination of the foregoing embodiments, the second metal may comprise one or more group 5 to group 11 transition metals.

In any one or combination of the foregoing embodiments, the first metal may comprise any one of the commonly used metals for metal oxide semiconductors, including aluminum, bismuth, cadmium, cerium, chromium, cobalt, copper, iron, gallium, indium, molybdenum, niobium, tantalum, tin, titanium, tungsten, vanadium or zinc.

In any one or combination of the foregoing embodiments, the first metal may comprise tin and the second metal comprises copper.

In some embodiments, a gas sensor comprises the gas-sensing element of any one or combination of the foregoing embodiments disposed between electrodes connected by a voltage-measuring circuit, current-measuring circuit, resistance-measuring circuit, impedance-measuring circuit, or conductance-measuring circuit.

In some embodiments, the resistance-measuring circuit of the gas sensor comprises a signal processor calibrated to determine hydrogen sulfide concentration based on measured resistance at the gas-sensing surface.

In a second aspect according to claim 8 a method of using the gas sensor of any one or combination of the foregoing embodiments comprises exposing the gas-sensing surface to a gas to be tested, and measuring resistance of the gas-sensing element between the electrodes to determine a presence or concentration of a gas component.

In any one or combination of the foregoing embodiments, the gas sensor may test for or may be configured to test for hydrogen sulfide.

In a third aspect according to claim 9, a method of making a gas-sensing element comprises disposing a transition metal dopant comprising a second metal at a surface of a semiconductor that is a metal oxide of a first metal, and disposing a metal chalcogenide on top of the doped surface, wherein the method comprises alternately depositing second metal and metal oxide semiconductor of the first metal to form a plurality of alternating deposits of second metal and metal oxide semiconductor of the first metal between the body and the doped surface.

### BRIEF DESCRIPTION OF THE DRAWINGS

The foregoing and other features, and advantages of the present disclosure are apparent from the following detailed description taken in conjunction with the accompanying drawings in which:
FIG. 1 is a schematic depiction of a cross-section view of an example embodiment of a gas-sensing element;
FIG. 2 is a schematic depiction of a cross-section view of another example embodiment of a gas-sensing element;
FIG. 3 is a schematic depiction of a gas sensor;
FIGS. 4A, 4B, and 4B are plots of gas sensor outputs of tested sensing elements; and
FIGS. 5A and 5B are plots of gas sensor outputs of tested sensing elements.

### DETAILED DESCRIPTION

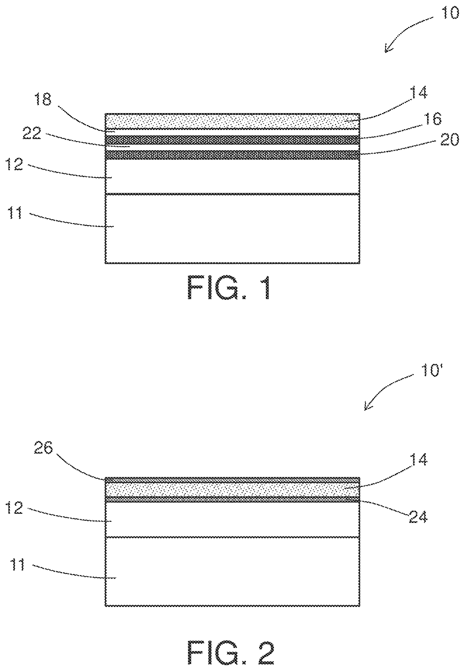

With reference now to the Figures, FIGS. 1 and 2 schematically depict an cross-section view of an example embodiment of a gas-sensing element. As shown in FIG. 1, gas-sensing element 10, 10' includes a metal oxide semiconductor body 12 disposed on a substrate having a gas-sensing surface 14 that comprises the metal oxide semiconductor material and a transition metal dopant. Typically, the gas-sensing elements 10, 10' are disposed on a substrate 11 as illustrated in FIGS. 1 and 2. Examples of metal oxide semiconductors include but are not limited to aluminum (III) oxide, bismuth (III) oxide, cadmium oxide, cerium (IV) oxide, chromium (III) oxide, cobalt (III) oxide, copper (II) oxide, iron (III) oxide, gallium (III) oxide, Indium (III) oxide, molybdenum (VI) oxide, niobium (V) oxide, nickel (II) oxide, tantalum (V) oxide, tin (IV) oxide, titanium (IV) oxide, tungsten (VI) oxide, vanadium (5) oxide, zinc (II) oxide and mixtures of these. Mixed metal oxides (e.g., SnO₂-CuO or other mixed oxides of the above metal oxides) can also be utilized, and the term "first metal" as used herein includes metal mixtures. Transition metal dopants are used to enhance the responsiveness of the metal oxide semiconductor to target gases being sensed for, such as hydrogen sulfide, and to allow for the target gas to be distinguished from other gases that may also produce a change in electrical resistance at the gas-sensing surface 14. In some embodiments, the dopant is a group 5 to group 11 transition metal. Examples of transition metal dopants include copper, silver, gold, iron, ruthenium, nickel, platinum, palladium, or vanadium. Although any of the above materials can exhibit a change in electrical resistance in response to exposure to various test gas components, the use of some materials for particular applications has been more widespread than other materials. For example, copper-doped tin oxide can be used for hydrogen sulfide sensing elements and platinum and palladium doping is commonly used in sensing for hydrogen or hydrocarbons. Such combinations and others are included within this disclosure. Various other materials can be included in the metal oxide semiconductor at the gas-sensing surface 14, including but not limited to noble metals (e.g., silver, gold). Dopants, metal oxide semiconductors, other materials, and combinations thereof are disclosed in Kaur, M. Aswal, D.K. and Yakhmi, J.V." Chemiresistor Gas Sensors: Materials, Mechanisms and Fabrication" Chapter 2 in , Science and Technology of Chemiresistor Gas Sensors, Ed. Aswal, D.K. and Gupta, S.K. Nova Science Publishers, New York, 2007., and in Bochenkov, V.E. and Sergeev, G.B. "Sensitivity, Selectivity, and Stability of Gas-Sensitive Metal-Oxide Nanostructures" Chapter 2, in Metal Oxide Nanostructures and Their Applications., American Scientific Publishers, California, 2010.

As mentioned above, the gas-sensing element includes an auxiliary component comprising a metal chalcogenide disposed at the gas-sensing surface that stabilizes the second metal at the gas-sensing surface. An example embodiment of internally-disposed second metal 16 between the metal oxide semiconductor body 12 and the gas-sensing surface (not claimed) is schematically depicted in FIG. 1. The element may also include metal oxide semiconductor 18 that is free of second metal (e.g., high purity metal oxide semiconductor) disposed between the internally disposed second metal 16 and the second metal-doped gas-sensing surface 14. In the claimed invention, the sensing element includes a plurality of deposits of second metal alternating with deposits of metal oxide semiconductor, as illustrated in FIG. 1 with additional second metal 20 and additional metal oxide semiconductor 22. Four deposits are illustrated in FIG. 1, but larger numbers (e.g., more than 10) of such alternating deposits can also be used.

Deposition of second metal or metal oxide semiconductor onto the metal oxide semiconductor body can be performed using thermal deposition techniques such as sputtering, physical vapor deposition, chemical vapor deposition, or thermal spray. Alternatively, any or all of the deposits can be grown layer by layer, for example, using solution-based epitaxy techniques such as sol-gel processing to form the individual layers. The term "layer" as used herein means any deposit of material, including islands and partial layers, as well as contiguous layers of material. Layers of internally-disposed second metal can range in thickness from 0 (meaning no contiguous layer such as where areas (e.g., islands) of deposited second metal having thicknesses as low as the mass equivalent of 0.2 Angstroms) to 20 nm. Layers of internally-disposed metal oxide semiconductor, which can be interspersed with deposits of the second metal, can range in thickness from 1 to 60 nm.

As mentioned above, the gas-sensing element includes an auxiliary component comprising a metal chalcogenide disposed at the gas-sensing surface that stabilizes the second metal at the gas-sensing surface. An example embodiment of a metal chalcogenide disposed at the gas-sensing surface or internally disposed in the gas-sensing element between the body and the gas-sensing surface adjacent to the gas-sensing surface is schematically depicted in FIG. 2. As shown in FIG. 2, gas-sensing element 10' includes metal oxide semiconductor body 12 and doped metal oxide semiconductor gas-sensing surface 14. In some embodiments, the metal chalcogenide can be applied internal to the gas-sensing element adjacent to the gas-sensing surface 14, as depicted by metal chalcogenide 24 in FIG. 2. In the claimed invention, the metal chalcogenide is applied over the gas-sensing surface, as depicted by metal chalcogenide 26 in FIG. 2. In some embodiments, the metal chalcogenide can be disposed (not shown) in the gas-sensing surface 14. In some embodiments, the metal chalcogenide can be disposed in a combination of more than one of the specified locations (e.g., both over and under the gas-sensing surface 14, or mixed in with and underneath the gas-sensing surface 14). In some embodiments (not shown), the auxiliary component can include both (1) internally-disposed second metal disposed in the gas-sensing element between the body and the gas-sensing surface, and (2) a metal chalcogenide disposed at the gas-sensing surface that stabilizes the second metal at the gas-sensing surface. For example, a gas-sensing element could have a metal chalcogenide over (24) the gas-sensing surface 14 and second metal disposed 16, 20 between the gas-sensing surface 14 and the metal oxide semiconductor body 12.

Examples of chalcogens for the metal chalcogenide include sulfur, selenium and tellurium. In some embodiments, the chalcogen is a chalcogen having a higher number on the periodic table than oxygen. Metals for the metal chalcogenide include but are not limited to silver, lead, zinc, iron, cadmium or other metals that provide a stable chalcogenide at the operating temperature of the sensing element. In some embodiments, the metal chalcogenide comprises a metal sulfide. Examples of metal sulfides include but are not limited to silver sulfide, lead sulfide, zinc sulfide, iron sulfide or cadmium sulfide. In some embodiments, the metal chalcogenide comprises silver sulfide. The metal chalcogenide can be introduced by applying the metal (e.g., silver, lead, zinc, iron) below or above the gas-sensing surface 14 using sputtering or any of the techniques referenced above for application of second metal 16 or metal oxide semiconductor 18, reacting with a reactive chalcogenide such as hydrogen sulfide, and sintering. Sintering may promote spreading of the metal chalcogenide through the gas-sensing surface 14.

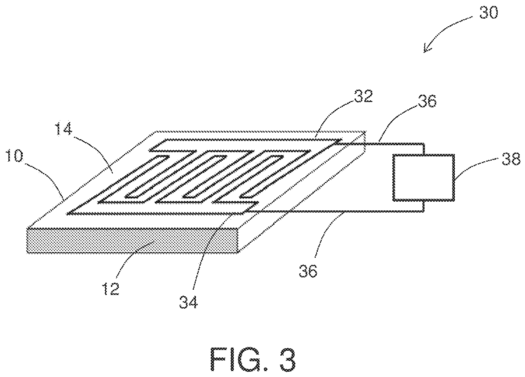

The above-described sensing element can be incorporated into a sensor 30 as schematically depicted in FIG. 3. As shown in FIG. 3, gas sensor 30 comprises the gas-sensing element 10 with metal oxide semiconductor body 12 and gas-sensing surface 14, integrated with either parallel or interdigitated (as shown, for higher gain) electrodes 32 and 34 configured to have doped metal oxide semiconductor at the gas-sensing surface 14 disposed between the interdigitated electrodes 32 and 34. The electrodes 32, 34 are depicted on top of the sensing element 10, but can also be disposed at the bottom. The electrodes are connected externally to the gas-sensing element 10 by an electrical circuit 36 that includes a signal processor 38. Signal processor 38 can be a voltmeter or ampere meter, but in many cases comprises a potentiostatic circuit, voltage divider circuit, bridge circuit, microprocessor, electronic control unit (ECU), or similar electronic device with integrated voltage and or amperage measurement functions and also can apply a voltage bias between the electrodes 32 and 34. Other sensor components including but not limited housings, mounting hardware, gas flow conduits, fluid chambers are not shown in FIG. 3, but can be incorporated into the sensor by the skilled person.

Additional disclosure is provided in the following Examples:

### EXAMPLES

As demonstrated by the following non-limiting example embodiments, some embodiments can provide a technical effect that promotes gas sensor stability and can mitigate gas sensor drift.

### Example 1

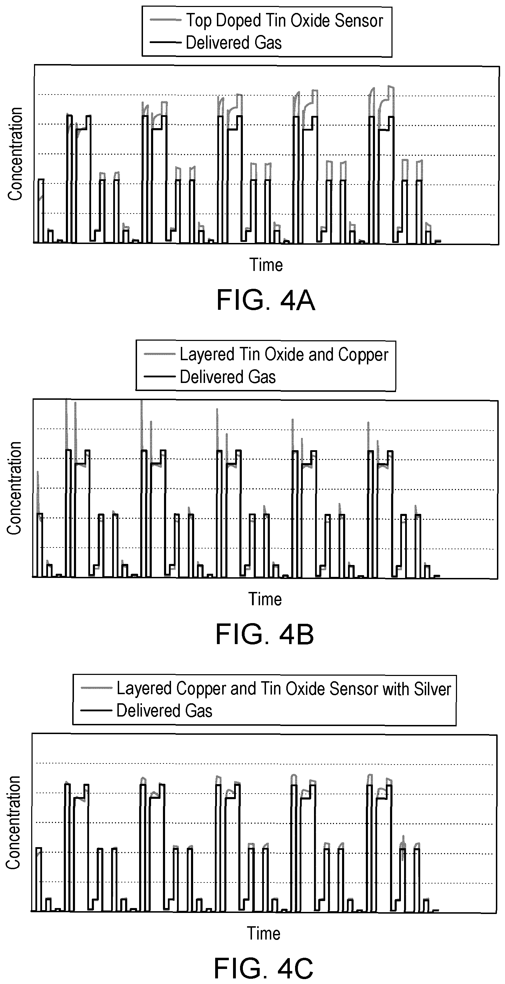

This Example is directed to disposing second metal between the gas-sensing surface of a sensing element and its metal oxide semiconductor body. Sensing elements were prepared by doping a tin oxide semiconductor surface with copper deposited by physical deposition means. Sensing element A was prepared as a control with the copper and gold dopants deposited onto the surface of a tin oxide body. Sensing element 1 was prepared by depositing copper and tin oxide in alternating layers to a tin oxide body, finishing with copper. Sensing element 2 was prepared similar to sensing element 1, except that silver was deposited and sulfided after the top copper dopant application. All three sensors were sintered. The sensing elements were exposed to varying concentrations of hydrogen sulfide over time, and the sensor output was recorded in measured hydrogen sulfide content. The results are shown in FIGS. 4A (sensing element A), 4B (sensing element 1), and 4C (sensing element 2). FIGS. 4A, 4B, and 4C depict overlaying plot of delivered concentration of hydrogen sulfide and the gas sensor output result in measured hydrogen sulfide content. The plotted sensor output in Figure 4A is typical of hydrogen sulfide sensor experiencing a phenomenon known as "sleeping", where the sensor response to hydrogen sulfide is initially reduced, but grows stronger with each exposure. In contrast, Figure 4B shows a longer term stabilizing effect of the layered sensor with improved stability and reduced sleep effect. In Figure 4C, it is seen that with the silver co-catalyst at the surface produces a refined response, also with improved stability and reduced sleep effect.

### Example 2

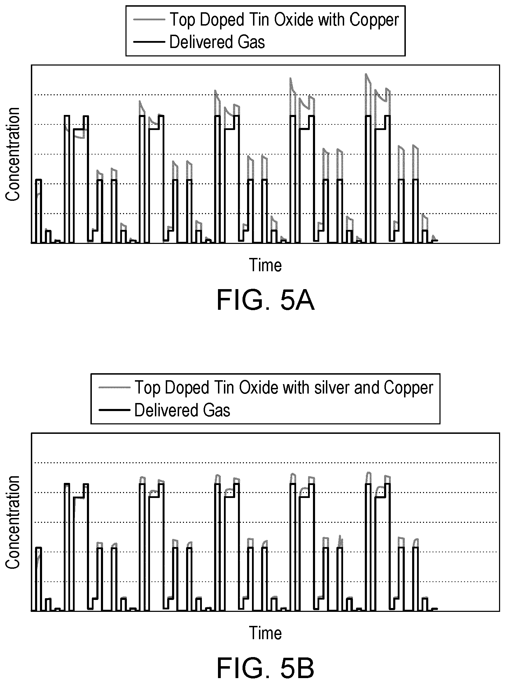

This Example is directed to disposing a metal chalcogenide at the gas-sensing surface of a surface-doped metal oxide conductor sensing element and its metal oxide semiconductor body. Sensing elements were prepared as in Example 1 by doping a tin oxide semiconductor surface with copper deposited by sputtering. Sensing element B was prepared as a control with the copper dopant deposited onto the surface of a tin oxide body. Sensing element 3 was prepared by depositing silver onto a copper top doped tin oxide body, followed by reaction with hydrogen sulfide to convert the silver to silver sulfide. The sensing elements were exposed to varying concentrations of hydrogen sulfide over time, and the sensor output was recorded in measured hydrogen sulfide content. The results are shown in FIGS. 5A (sensing element A) and 5B (sensing element 3). FIGS. 5A and 5B depict overlaying plot of delivered concentration of hydrogen sulfide and the gas sensor output result in measured hydrogen sulfide content. The plotted sensor output in Figure 5A is typical of hydrogen sulfide sensor experiencing a phenomenon known as "sleeping", where the sensor response to hydrogen sulfide is initially reduced, but grows stronger with each exposure. In contrast, Figure 5B shows a longer term stabilizing effect of the layered sensor with improved stability reduced sleep effect.

## Claims

1. A gas-sensing element (10), comprising:
a body (12) comprising a semiconductor that is a metal oxide of a first metal;
a gas-sensing surface (14) over the body, comprising metal oxide semiconductor of the first metal and a dopant comprising a second metal that is a transition metal and is different than the first metal; and
an auxiliary component comprising a metal chalcogenide disposed at the gas-sensing surface;
wherein the sensing element comprises a plurality of alternating deposits of the metal oxide semiconductor of the first metal and deposits of the second metal, disposed in the gas-sensing element between the body and the gas-sensing surface.

2. The gas-sensing element of claim 1, wherein the auxiliary component stabilizes the second metal at the gas-sensing surface.

3. The gas-sensing element of claim 2, wherein the metal chalcogenide comprises a metal sulfide.

4. The gas-sensing element of any of claims 1-3, wherein the second metal comprises one or more group 5 to group 11 transition metals.

5. The gas-sensing element of any of claims 1-4, wherein the first metal comprises aluminum, bismuth, cadmium, cerium, chromium, cobalt, copper, iron, gallium, indium, molybdenum, niobium, tantalum, tin, titanium, tungsten, vanadium or zinc.

6. The gas-sensing element of any of claims 1-5, wherein the first metal comprises tin and the second metal comprises copper.

7. A gas sensor comprising the gas-sensing element of any of claims 1-6 disposed between electrodes connected by a voltage-measuring circuit, current-measuring circuit, resistance-measuring circuit, impedance-measuring circuit, or conductance-measuring circuit, preferably wherein the resistance-measuring circuit comprises a signal processor calibrated to determine hydrogen sulfide concentration based on measured resistance at the gas-sensing surface.

8. A method of using the gas sensor of claim 7, comprising exposing the gas-sensing surface to a gas to be tested, and measuring resistance of the gas-sensing element between the electrodes to determine a presence or concentration of a gas component, preferably wherein the gas component comprises hydrogen sulfide.

9. A method of making a gas-sensing element, comprising disposing a transition metal dopant comprising a second metal that is a transition metal at a surface of a semiconductor that is a metal oxide of a first metal, and disposing a metal chalcogenide on top of the doped surface, wherein the method comprises alternately depositing second metal and metal oxide semiconductor of the first metal to form a plurality of alternating deposits of second metal and metal oxide semiconductor of the first metal between the body and the doped surface.

## Patentansprüche

1. Gaserfassungselement (10), umfassend:
einen Körper (12), umfassend einen Halbleiter, der ein Metalloxid eines ersten Metalls ist:
eine Gaserfassungsoberfläche (14) über dem Körper, umfassend Metalloxidhalbleiter des ersten Metalls und einen Dotierstoff, umfassend ein zweites Metall, das ein Übergangsmetall und vom ersten Metall verschieden ist; und
eine Hilfskomponente, umfassend ein Metallchalcogenid, das an der Gaserfassungsoberfläche angeordnet ist;
wobei das Erfassungselement eine Vielzahl von abwechselnden Abscheidungen des Metalloxidhalbleiters des ersten Metalls und Abscheidungen des zweiten Metalls umfasst, die in dem Gaserfassungselement zwischen dem Körper und der Gaserfassungsoberfläche angeordnet sind.

2. Gaserfassungselement nach Anspruch 1, wobei die Hilfskomponente das zweite Metall an der Gaserfassungsoberfläche stabilisiert.

3. Gaserfassungselement nach Anspruch 2, wobei das Metallchalcogenid ein Metallsulfid umfasst.

4. Gaserfassungselement nach einem der Ansprüche 1-3, wobei das zweite Metall eines oder mehrere Übergangsmetalle der Gruppe 5 bis Gruppe 11 umfasst.

5. Gaserfassungselement nach einem der Ansprüche 1-4, wobei das erste Metall Aluminium, Bismut, Cadmium, Cer, Chrom, Cobalt, Kupfer, Eisen, Gallium, Indium, Molybdän, Niob, Tantal, Zinn, Titan, Wolfram, Vanadium oder Zink umfasst.

6. Gaserfassungselement nach einem der Ansprüche 1-5, wobei das erste Metall Zinn umfasst und das zweite Metall Kupfer umfasst.

7. Gassensor, umfassend das Gaserfassungselement nach einem der Ansprüche 1-6, das zwischen Elektroden angeordnet ist, die durch eine Spannungsmessschaltung, Strommessschaltung, Widerstandsmessschaltung, Impedanzmessschaltung, oder Leitfähigkeitsmessschaltung verbunden sind, wobei die Widerstandsmessschaltung vorzugsweise einen Signalprozessor umfasst, der kalibriert ist, um eine Schwefelwasserstoffkonzentration basierend auf einem gemessenen Widerstand an der Gaserfassungsoberfläche zu bestimmen.

8. Verfahren zum Verwenden des Gassensors nach Anspruch 7, umfassend ein Aussetzen der Gaserfassungsoberfläche einem zu prüfenden Gas und Messen des Widerstands des Gaserfassungselements zwischen den Elektroden zum Bestimmen eines Vorhandenseins oder einer Konzentration einer Gaskomponente, wobei die Gaskomponente vorzugsweise Schwefelwasserstoff umfasst.

9. Verfahren zum Herstellen eines Gaserfassungselements, umfassend ein Anordnen eines Übergangsmetalldotierstoffs, umfassend ein zweites Metall, das ein Übergangsmetall an einer Oberfläche eines Halbleiters ist, der ein Metalloxid eines ersten Metalls ist, und Anordnen eines Metallchalcogenids oben auf der dotierten Oberfläche, wobei das Verfahren abwechselnd Abscheiden von zweitem Metall und Metalloxidhalbleiter des ersten Metalls umfasst, um eine Vielzahl von abwechselnden Abscheidungen von zweitem Metall und Metalloxidhalbleiter der ersten Metalls zwischen dem Körper und der dotierten Oberfläche zu bilden.

## Revendications

1. Élément de détection de gaz (10), comprenant :
un corps (12) comprenant un semi-conducteur qui est un oxyde métallique d'un premier métal ;
une surface de détection de gaz (14) sur le corps, comprenant un oxyde métallique semi-conducteur du premier métal et un dopant comprenant un second métal qui est un métal de transition et qui est différent du premier métal ; et
un composant auxiliaire comprenant un chalcogénure de métal disposé au niveau de la surface de détection de gaz ;
dans lequel l'élément de détection comprend une pluralité de dépôts alternés de l'oxyde métallique semi-conducteur du premier métal et de dépôts du second métal, disposés dans l'élément de détection de gaz entre le corps et la surface de détection de gaz.

2. Élément de détection de gaz selon la revendication 1, dans lequel le composant auxiliaire stabilise le second métal au niveau de la surface de détection de gaz.

3. Élément de détection de gaz selon la revendication 2, dans lequel le chalcogénure de métal comprend du sulfure métallique.

4. Élément de détection de gaz selon l'une quelconque des revendications 1-3, dans lequel le second métal comprend un ou plusieurs métaux de transition du groupe 5 au groupe 11.

5. Élément de détection de gaz selon l'une quelconque des revendications 1-4, dans lequel le premier métal comprend de l'aluminium, du bismuth, du cadmium, du cérium, du chrome, du cobalt, du cuivre, du fer, du gallium, de l'indium, du molybdène, du niobium, du tantale, de l'étain, du titane, du tungstène, du vanadium ou du zinc.

6. Élément de détection de gaz selon l'une quelconque des revendications 1-5, dans lequel le premier métal comprend de l'étain et le second métal comprend du cuivre.

7. Capteur de gaz comprenant l'élément de détection de gaz selon l'une quelconque des revendications 1-6 disposé entre des électrodes connectées par un circuit de mesure de tension, un circuit de mesure de courant, un circuit de mesure résistance, un circuit de mesure d'impédance ou un circuit de mesure de conductance, de préférence dans lequel le circuit de mesure de résistance comprend un processeur de signal étalonné pour déterminé une concentration en sulfure d'hydrogène sur la base de la résistance mesurée au niveau de la surface de détection de gaz.

8. Procédé d'utilisation du capteur de gaz selon la revendication 7, comprenant l'exposition de la surface de détection de gaz à un gaz à tester, et la mesure de la résistance de l'élément de détection de gaz entre les électrodes pour déterminer une présence ou une concentration d'un composant gazeux, de préférence dans lequel le composant gazeux comprend du sulfure d'hydrogène.

9. Procédé de fabrication d'un élément de détection de gaz, comprenant la disposition d'un dopant de métal de transition comprenant un second métal qui est un métal de transition au niveau d'une surface d'un semi-conducteur qui est un oxyde métallique d'un premier métal, et la disposition d'un chalcogénure de métal sur la surface dopée, dans lequel le procédé comprend le dépôt alterné du second métal et de l'oxyde métallique semi-conducteur du premier métal pour former une pluralité de dépôts alternés du second métal et de l'oxyde métallique semi-conducteur du premier métal entre le coprs et la surface dopée.
